(19) Europäisches Patentamt
European Patent Office
Office européen des brevets

(11) **EP 2 688 859 B1**

(12) **EUROPÄISCHE PATENTSCHRIFT**

(45) Veröffentlichungstag und Bekanntmachung des Hinweises auf die Patenterteilung:
**21.11.2018 Patentblatt 2018/47**

(51) Int Cl.:
*C07C 1/20* (2006.01)     *C07C 4/06* (2006.01)
*C07C 11/04* (2006.01)     *C07C 11/16* (2006.01)

(21) Anmeldenummer: **12703778.6**

(22) Anmeldetag: **07.02.2012**

(86) Internationale Anmeldenummer:
**PCT/EP2012/052040**

(87) Internationale Veröffentlichungsnummer:
**WO 2012/126670 (27.09.2012 Gazette 2012/39)**

(54) **VERFAHREN UND ANLAGE ZUR HERSTELLUNG VON NIEDERMOLEKULAREN OLEFINEN**

PROCESS AND PLANT FOR PREPARATION OF LOW MOLECULAR WEIGHT OLEFINS

PROCÉDÉ ET INSTALLATION POUR LA PRÉPARATION D'OLÉFINES DE BAS POIDS MOLÉCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.03.2011 DE 102011014892**

(43) Veröffentlichungstag der Anmeldung:
**29.01.2014 Patentblatt 2014/05**

(73) Patentinhaber: **Air Liquide Global E&C Solutions Germany GmbH**
**60439 Frankfurt am Main (DE)**

(72) Erfinder:
• **BIRKE, Gerhard**
**60389 Frankfurt (DE)**
• **BACH, Hermann**
**56412 Heiligenroth (DE)**

(74) Vertreter: **Keil & Schaafhausen**
**Patent- und Rechtsanwälte PartGmbB**
**Friedrichstraße 2-6**
**60323 Frankfurt am Main (DE)**

(56) Entgegenhaltungen:
**EP-A1- 2 058 290     DE-A1-102006 026 103**

## Beschreibung

Die vorliegende Erfindung betrifft ein Verfahren zur Herstellung von kurzkettigen oder niedermolekularen Olefinen, insbesondere von Ethylen und Propylen, wobei ein wenigstens ein Oxygenat enthaltender Eduktstrom und ein wenigstens ein $C_{4+}$ -Olefin enthaltender Eduktstrom simultan in wenigstens einem gleichen Reaktor an einem gleichen Katalysator zu einem niedermolekularen Olefine und Benzinkohlenwasserstoffe umfassenden Produktgemisch umgesetzt werden. Des Weiteren betrifft die vorliegende Erfindung auch eine zur Durchführung des Verfahrens geeignete Anlage.

Propen ($C_3H_6$), oft auch als Propylen bezeichnet, ist einer der wichtigsten Ausgangsstoffe der chemischen Industrie. Die Nachfrage nach dem Grundstoff Propylen steigt weltweit, wobei Propylen genauso wie Ethylen zumeist in einem Steam-Cracker in einem vom Verfahren und den Rohstoffen abhängigen Verhältnis aus Erdöl erzeugt wird.

Um zusätzliches Propylen zu gewinnen, existieren eine Reihe von Verfahren, wie etwa der PDH-Prozess, der von Propan als Edukt ausgeht. Da jedoch nach wie vor durch Steam-Cracking der größte Anteil an Propylen erzeugt wird (etwa 70 %), gibt es eine Tendenz, die in Crackern oder anderen petrochemischen Anlagen anfallenden $C_4$- bis $C_8$ -Olefine zu zusätzlichem Propylen, teilweise auch zu Ethylen zu konvertieren.

Dies kann zum einen über das Methatese-Verfahren erfolgen, welches auf einer Synproportionierung von Ethylen und Butylen beruht. Nachteilig ist hier, dass dazu die Ethylen-Produktion aufgestockt werden muss und nur $C_4$ -Olefine konvertiert werden können.

Weiterhin ist eine Olefin-Konversion möglich, bei der $C_{4+}$ -Olefine zu Propylen umgesetzt werden. Diese Spaltung erfolgt mittels des Propylur- oder des OCP-Verfahrens und wird vor allem genutzt, um die in einer Cracker-Anlage produzierten - vergleichsweise niedrig zu bewertenden - $C_{4+}$ -Olefine zur Produktion von Propylen zu nutzen. Aufgrund der Endothermie der Reaktion fällt die Temperatur im Reaktor jedoch mit zunehmendem Umsatz und begrenzt somit die erreichbare Propylenausbeute.

Schließlich bietet sich das Methanol-To-Propylene-Verfahren (auch MTP®-Verfahren) an, bei dem Methanol/Dimethylether oder auch andere Oxygenate an einem zumeist zeolithischen Katalysator zu Propylen umgesetzt werden.

Die DE 10 2005 048 931 beschreibt ein solches MTP®-Verfahren zur Herstellung von $C_2$- bis $C_4$- Olefinen aus einem Wasserdampf und Oxygenate, wie Methanol und/oder Dimethylether, enthaltenden Eduktgemisch, bei dem das Eduktgemisch in wenigstens einem Reaktor durch eine heterogen-katalysierte Reaktion zu einem niedermolekulare Olefine und Benzinkohlenwasserstoffe umfassenden Reaktionsgemisch umgesetzt wird. Dieses Reaktionsgemisch wird dann in einer ersten Trenneinrichtung in ein $C_{5-}$ -olefinreiches Gemisch, eine an $C_{5+}$ -Benzinkohlenwasserstoffen reiche Fraktion und eine wässrige Phase aufgetrennt. Die $C_{5+}$ -Benzinkohlenwasserstoff reiche Fraktion wird danach einer zweiten Trenneinrichtung zugeführt, in der die Aromaten aus dem Gemisch entfernt werden. Der verbleibende, weitgehend aromatenfreie Reststrom wird als Recyclingstrom wenigstens teilweise in den Reaktor rückgeführt. Dies hat den Vorteil, dass sich der Olefin-Anteil größtenteils zu Propylen umwandeln lässt, wodurch die Ausbeute an Propylen insgesamt erhöht wird.

Die WO 2008/039552 A1 lehrt ein Verfahren, in dem MTP®- und Spaltverfahren hintereinander geschaltet sind. Dazu werden die Oxygenate in einem Reaktor in Olefine nach dem MTP®-Verfahren umgewandelt. Aus dem so erhaltenen Produktstrom werden Ethylen und Propylen abgetrennt. Bei dieser Abtrennung werden auch verbleibende Oxygenate und Wasser aus dem Strom entfernt, wodurch eine reine $C_{4+}$ -Fraktion entsteht, die in einen Olefinspaltreaktor überführt wird. Durch Spaltung der Olefine kann dann noch weiteres Ethylen und Propylen gewonnen werden.

Die US 2004/008 7824 A1 beschreibt eine Kombination der beiden Verfahren zur Umsetzung von Oxygenaten und Spaltung von Olefinen ausgehend von dem Produktgemisch aus einer Fischer-Tropsch-Synthese. Dazu wird der Produktstrom, welcher sowohl Oxygenate als auch $C_{6+}$ -Olefine enthält, mit einem sauren, Olefin-spaltenden Katalysator in Verbindung gebracht. Sowohl die Oxygenate als auch die höherwertigen Olefine werden dann zu leichten Olefinen wie Propylen, Buten und Penten umgesetzt. Die Umsetzung erfolgt bei Temperaturen zwischen 260 und 454° C sowie einem Druck unterhalb von 69 bar. Durch diese Reaktionsbedingungen entsteht jedoch noch immer eine erhebliche $C_{4+}$ -Olefin-Fraktion, wohingegen $C_4$/$C_5$ Olefine nicht nennenswert zu leichteren Olefinen umgesetzt werden und das Wertprodukt Ethylen kaum gewonnen werden kann.

Den Verbund einer MTP®- mit einer Cracker-Anlage beschreibt die DE 10 2007 045 238 A1, wobei MTP®-Reaktor und Cracker parallel geschaltet sind. Die jeweiligen Zwischenproduktströme des Steam-Crackers und des Reaktors werden zumindest teilweise zusammengeführt. Dies hat den Vorteil, dass die sich anschließenden Trenneinrichtungen gemeinsam genutzt werden können. Teile dieser Ströme werden zudem zu dem Steam-Cracker und/oder dem MTP®-Reaktor zurückgeführt, wodurch sich insbesondere im MTP®-Reaktor die längerkettigen Alkene zu leichteren Olefinen, insbesondere Ethylen und Propylen, spalten.

Mit einer Verschaltung nach der DE 10 2007 045 238 A1 können aus 1.660.000 jato Methanol im Verbund von MTP®- und Cracker-Anlagen 50.899 jato Ethylen und 440.331 jato Propylen mehr als in der reinen Cracker-Anlage produziert und damit das Verhältnis $C_2$ zu $C_3$ zu Gunsten von Propylen wirksam von 43,06 auf 1,86 verschoben werden.

Allerdings liegt die Selektivität der Summe der Wertprodukte Propylen und Ethylen bezogen auf Methanol nur bei 0,68 und damit deutlich < 1, woraus sich ergibt, dass die Selektivität nur geringfügig größer ist als in einer autarken MTP®-Anlage. Dies bedeutet, dass die erhöhte Ausbeute an Propylen hauptsächlich der reinen Nebeneinanderschaltung von MTP®- und Cracker-Anlage zuzuordnen ist und keine wesentliche Reduktion der im MTP®-/ Cracker-Komplex vorhandenen Olefine mit höheren C-Zahlen erfolgen konnte.

Die EP 2 058 290 A1 beschreibt ein Verfahren zur Herstellung von Propylen, wobei einem Reaktor eine Mischung aus $C_{4-}$ bis $C_{5-}$Olefinen, Dimethylether und/oder Methanol zugeführt wird. Das molare Verhältnis der zugeführten Olefine zu dem zugeführten Dimethylether und/oder dem zugeführten Methanol soll zwischen 0,25 und 7,5 liegen, was bedeutet, dass die Summe aller Produkte aus Kohlenstoffzahl und Molenfluss der Olefine geteilt durch die Summe der Produkte von Kohlenstoffzahl und Molenfluss von Dimethylether und Methanol in diesem Verhältnis liegt.

Die DE 10 2006 026 103 A1 beschreibt einen Reaktor zur Herstellung von $C_{2-}$ bis $C_{8-}$ Olefinen aus einem gasförmigen Oxygenat, Wasser und einem oder mehreren Olefinen. Dieser ist so ausgestaltet, dass der von oben kommende Stoffstrom aus den genannten Edukten mehrere Reaktionsstufen durchströmt, die jeweils aus einem Tragboden mit einer darauf befindlichen Katalysatorschicht ausgebildet sind.

Aus der DE 10 2007 045 238 A1 ist auch bekannt, dass durch die Gegenwart von längerkettigen Olefinen und damit verbunden dem Ablauf endothermer Reaktionen die Temperatur im Reaktor gesenkt werden kann. Allerdings ist dem Stand der Technik nicht zu entnehmen, in welchem Verhältnis die Ströme von Oxygenaten und $C_{4+}$ -Olefinen stehen müssen, um die Temperatur im Reaktor auf einem nahezu konstanten Niveau zu halten. Eine möglichst gleichmäßige Temperaturführung ist jedoch deshalb wichtig, weil die Olefin-Spaltung bisher dadurch begrenzt wird, dass die endotherme Reaktion die Temperatur im Reaktor absinken lässt, so dass schließlich nicht mehr genügend Energie zum Überwinden der Aktivierungsenergie der Reaktion zur Verfügung steht und die Reaktion deshalb nicht vollständig erfolgt. Gleichzeitig ist das klassische MTP®-Verfahren hoch exotherm, so dass trotz aufwendiger Kühlkonstruktion die ansteigende Temperatur im Reaktor die Selektivität hinsichtlich Propylen verringert.

Es ist daher Aufgabe der Erfindung, ein Verfahren bereit zu stellen, bei dem durch eine möglichst homogene Temperaturführung eine Maximierung der Ausbeute an Propylen und Ethylen erreicht werden kann.

Diese Aufgabe wird mit der Erfindung mit den Merkmalen des Anspruchs 1 im Wesentlichen dadurch gelöst, dass ein wenigstens ein Oxygenat enthaltender Eduktstrom und ein wenigstens ein $C_{4+}$-Olefin enthaltender Eduktstrom simultan in wenigstens einem gleichen Reaktor an einem gleichen Katalysator zu einem niedermolekulare Olefine und Benzinkohlenwasserstoffe umfassenden Produktgemisch umgesetzt wird. Das Verhältnis von Oxygenaten zu $C_{4+}$-Olefinen wird dabei erfindungsgemäß auf ein Verhältnis zwischen 0,05 und 0,5, vorzugsweise zwischen 0,15 und 0,3 eingestellt. Dieses Verhältnis berechnet sich nach der Formel

$$V = \frac{\sum\limits_{j} k_{Oxygenat-j} * n_{Oxygenat-j}}{\sum\limits_{i} k_{Olefin-i} * n_{Olefin-i} + \sum\limits_{j} k_{Oxygenat-j} * n_{Oxygenat-j}}$$

mit:

$k_{Oxygenat-j}$ : Kohlenstoff-Zahl des Oxygenats j
$n_{Oxygenat-j}$ : Molenstrom des Oxygenats j
$k_{Olefin-i}$ : Kohlenstoff-Zahl des Olefins i
$n_{Olefin-i}$ : Molenstrom des Olefins i

Da simultan aus den Oxygenaten in einer exothermen Reaktion leichte Olefine gebildet und die $C_{4+}$ -Olefine in einer endothermen Reaktion zu leichten Olefinen gespalten werden, halten sich Energiebedarf und Energiebereitstellung die Waage. Die Energietönung der Gesamtreaktion kann durch die optimierte Einstellung des Verhältnisses Olefine:Oxygenate kontrolliert und der Reaktor so bei optimaler Temperatur betrieben werden. Dadurch wird die Propylen-Ausbeute in Bezug auf die gewählten Druck- und Temperaturreaktionsbedingungen maximiert. Normalerweise wird dazu ein konstantes oder gegen Reaktorende leicht ansteigendes Temperaturprofil eingestellt, zum Beispiel ein Temperaturgradient von 480 zu 500°C

Aus dem erfindungsgemäßen Verfahren ergeben sich mehrere Synergieeffekte. Zum einen ist die Steigerung der Ausbeute der Wertprodukte Propylen und Ethylen ohne Ausbau der Kapazität der eigentlichen Cracker-Anlage zu nennen. Zum anderen wird die nachgeschaltete Hydrierung zur Umsetzung der längerkettigen Olefine entlastet, da der Anteil der längerkettigen Olefine gesenkt werden kann. Dieser Effekt kann insbesondere dann zum Tragen kommen, wenn eine Olefin-Trennung wie sie in der WO 2006/048184 A1 beschrieben ist, an eine entsprechende Anlage angeschlossen wird, und die olefinarmen Produktströme direkt zum Cracker zurückgeführt werden können. Schließlich kann es auch zu einer Steigerung der Aromatenausbeute kommen, was deshalb wirtschaftlich vorteilhaft sein kann, wenn es Ziel der Anlage ist, hauptsächlich höhere Aromaten zu erzeugen.

Eine Kopplung beider Grundreaktionen in dem erfindungsmäßigen Olefin-Konversionsverfahren führt wie beschrieben zu einer energetischen Kompensation. Zu diesem Zweck kann auf der einen Seite in eine bestehende MTP®-Anlage eine maximierte Menge an Fremdolefinen eingeführt werden oder es kann auf der anderen Seite von dem noch häufiger vorliegenden Fall ausgegangen werden, dass durch das Vorhandensein einer Cracker-Anlage Olefine verfügbar sind. Diese Olefine können mit minimiertem Einsatz von Methanol oder anderen Oxygenaten zu Propylen umgesetzt werden. Der Verbund einer Cracker- oder einer anderen Olefin-Nebenprodukte produzierenden Anlage mit einer erfindungsmäßigen Olefinkonversionsanlage erlaubt somit eine maßgeschneiderte und skalierbare Propylen-Produktion.

Der Reaktor und das Verfahren können darüber hinaus gegenüber dem klassischen MTP®-Verfahren stark vereinfacht werden. Zum einen hat der Reaktor nur 3 bis 4 Katalysatorbetten gegenüber 6 beim Standard-MTP® Reaktor. Die für die Verdampfungskühlung zwischen den Betten erforderlichen Einbauten entfallen ebenso wie ein dem MTP®-Reaktor vorgeschalteter DME-Reaktor mit nachfolgender Teilkondensation des Reaktionswassers.

Eine teilweise Rückführung von nicht umgesetzten Olefinen ermöglicht es, Einsatzströme mit unterschiedlichen Olefin-Gehalten zu verarbeiten oder unterschiedliche Mengen an Wertprodukten Propylen und Ethylen herstellen zu können. Dazu wird von dem Produktgemisch aus dem Reaktor in einer ersten Trenneinrichtung ein $C_{3-}$ -Olefin reiches Gemisch von einem an $C_{4+}$-Benzinkohlenwasserstoffen reichen Strom abgetrennt. Der benzinkohlenwasserstoffreiche Strom, der überwiegend Fraktionen mit einer Kettenlänge von mindestens vier Kohlenstoffatomen aufweist, wird danach einer zweiten Trenneinrichtung zugeführt und dort in einen, einen Großteil der $C_4$-Verbindungen enthaltenden Strom, eine Fraktion, die überwiegend Verbindungen mit wenigstens fünf C-Atomen aufweist, und einen Recyclingstrom aufgetrennt, wobei sich der Recyclingstrom vorzugsweise im Wesentlichen aus $C_4$-$C_6$-Verbindungen zusammensetzt. Der Recyclingstrom wird wenigstens teilweise in den Reaktor zurückgeführt, wobei das Molverhältnis zwischen dem Recyclingstrom und dem wenigstens ein $C_{4+}$-Olefin enthaltenden Eduktstrom zwischen 0,1 und 1,5 liegt.

In einer vorteilhaften Ausführungsform der Erfindung wird die zweite Trenneinrichtung bei einem Druck von 4-15 bar betrieben, und der Recyclingstrom wird über eine Leitung als Seitenabzug, vorzugsweise in Gasform, entnommen und unmittelbar in eine in den Reaktor mündende Leitung zurückgeführt.

Die Aufbereitung des Recyclingstroms hat eine große Bedeutung für die Propylen/ Ethylenausbeuten. Weil nur $C_{4+}$ Olefine sich in merklichem Ausmaß zu Propylen/ Ethylen umsetzen lassen, soll der Recyclingstrom eine Fraktion mil einem maximalen Olefinanteil enthalten. Abhängig von der Zusammensetzung des Eduktstroms C bezüglich der Olefine $C_4$ - $C_8$ enthält der Einsatzstrom B zur zweiten Trenneinrichtung unterschiedliche Restanteile an nicht umgesetzten $C_4$ - $C_6$ Olefinen. In der zweiten Trenneinrichtung werden diese möglichst gut im Recyclingstrom konzentriert, indem ein möglichst großer Anteil von $C_{4-6}$ Paraffinen in den entsprechenden Produktströmen A und F aus dem Kreislauf entfernt wird.

In einem einfachen Fall wird erfindungsgemäß die zweite Trenneinrichtung als Destillationskolonne ausgeführt, wobei der Recyclingstrom zwischen der Position des Einsatzstroms und dem Kolonnenkopf in einer geeigneten Position als Seitenabzug entnommen wird. Vorzugsweise wird die Kolonne bei einem Druck einige bar oberhalb des Reaktordrucks betrieben, damit der Seitenabzugsstrom als Gasstrom entnommen und ohne weitere Kompression zum Reaktor geführt werden kann. Das hat den Vorteil, dass eine Wiederverdampfung (im Fall eines flüssigen Recyclingstroms) bzw. eine Verdichtung (im Fall eines gasförmigen Recyclingstroms bei niedrigem Druck) mit einem Kompressor entfallen kann.

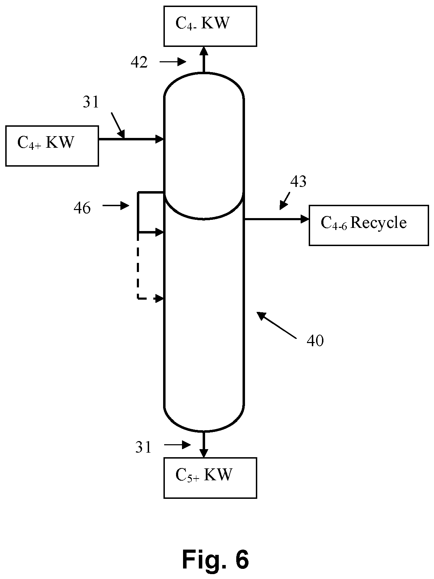

Typischerweise muss in einer Anlage mit schwankenden Zusammensetzungen des Eduktstroms C oder sogar mit unterschiedlichen Eduktströmen gerechnet werden. Damit ergeben sich im Einsatzstrom B zur zweiten Trenneinrichtung zeitlich unterschiedliche $C_{4-6}$ Trennaufgaben, die in der Standardschaltung mit Seitenabzug nur noch ungenügend zu bewältigen sind. Erfindungsgemäß ist unter diesen komplexeren Voraussetzungen eine integrierte Destillationskolonne nach Fig. 6 auszuführen. Diese ermöglicht es, im oberen Teil der Kolonne einen $C_4$-Schnitt so abzutrennen, dass im unteren Teil das Kopfprodukt, d.h. der Recyclingstrom R eine optimale Menge $C_4$ enthält. Im unteren Teil der Kolonne kann unabhängig davon eine optimale Menge $C_{5-6}$ in das Kopfprodukt, d.h. den Recyclingstrom R gelenkt werden. Der vom oberen in den unteren Teil geführte Strom kann als Rückfluss aufgegeben werden, wenn der Eduktstrom C hauptsächlich $C_4$ Kohlenwasserstoffe enthält. Wenn der Anteil von $C_5$ Kohlenwasserstoffen im Eduktstrom C dagegen hoch ist, ist der Strom als Einsatz in den mittleren Teil aufzugeben, damit eine Abreicherung von $C_5$ im Recyclingstrom R erfolgen kann.

Weiterhin muss erfindungsgemäß ein Katalysator eingesetzt werden, der sowohl in der Lage ist, die Umwandlung der Oxygenate zu niedermolekularen Olefinen zu katalysieren als auch als Spaltkatalysator für die $C_{4+}$ -Olefine wirkt. Erfindungsgemäß eignet sich dafür vor allem ein formselektives Zeolithmaterial, insbesondere ein Alumosilikat vom Pentasil-Typ ZSM-5.

Günstig ist auch, den Katalysator in mehreren, wenigstens zwei, vorzugsweise vier Katalysatorbetten in dem Reaktor einzuordnen und den Eduktstrom so in mehrere Teilströme einzuteilen, dass jeder Teilstrom auf eines der Katalysatorbetten geleitet wird. Dadurch kann eine Temperaturregelung jeder einzelnen Stufe des Reaktors durch die anteilige Aufgabe von gasförmigem Oxygenat zum Produktstrom der vorherigen Stufe bewirkt werden. Durch Variation der Menge an Oxygenat im Verhältnis zu den Kohlenwasserstoff/Olefin-Anteilen, der Aufgabetemperatur und den Reaktionsbedingungen, kann so die Austrittstemperatur jeder Stufe für ein weites Spektrum von Einsatzbedingungen eingestellt und geregelt werden.

Als Oxygenate eignen sich besonders Alkohole, vor allem Methanol, aber auch Gemische wie zum Beispiel Bioethanol mit seinen Verunreinigungen oder die in einer Bioethanolanlage als Nebenprodukte ("Fuselöl") anfallenden $C_3$- bis $C_6$-Alkohole. Das erfindungsgemäße Verfahren stellt somit eine seltene Verknüpfung der Rohstoffgewinnung aus fossilen Energieträgern und der Rohstoffgewinnung aus nachwachsenden Rohstoffen dar. Eine solche Verknüpfung ist insbesondere in Übergangssituationen von einem Energieträger zum anderen vorteilhaft und erlaubt es, Produktivitätsschwankungen in einem der beiden Verfahren auszugleichen.

Erfindungsgemäß wird der Druck am Eintritt des Reaktors auf einen Wert zwischen 1,5 bis 10 bar, vorzugsweise auf einen Wert zwischen 1,8 bis 5 bar, eingestellt. Dadurch kann sichergestellt werden, dass beide Reaktionen mit besonders hoher Selektivität hinsichtlich Propylen ablaufen.

Je höher der Druck, desto niedriger die Propylen-Ausbeute und umso weniger Oxygenat wird zur Einstellung der Temperatur benötigt. In einer Olefin-Überschusssituation bietet es sich also insbesondere an, bei niedrigerer Propylen-Ausbeute als der theoretisch möglichen den Druck auf 3 bis 5 bar anzuheben, um die Dimensionen der Apparate und damit die Kompressions- und Investitionskosten zu senken.

Die Temperatur am Austritt des Reaktors liegt zwischen 460 und 560 °C vorzugsweise zwischen 480 und 510 °C. Dieses Temperaturfenster ermöglicht die simultane Ausführung von MTP®-Verfahren und Spaltreaktionen ohne die Bildung unerwünschter Nebenprodukte.

Als Ausgangsstoff eignen sich insbesondere auch Oxygenate, die bei der Produktion von Ethanol durch Fermentation und/oder bei einer Fischer-Tropsch-Synthese als Nebenprodukt angefallen sind. Der $C_{4+}$-Olefine enthaltende Eduktstrom kann mindestens ein $C_2$ bis $C_{10}$-Olefin enthalten, das bei einer Fischer-Tropsch-Synthese als Primärprodukt erzeugt wurde. Auch hier findet sich wieder die vorteilhafte Verknüpfung von Verfahren mit Ausgangsstoffen sowohl aus dem Bereich der fossilen als auch der nachwachsenden Rohstoffe. Das gerade im Bereich der nachwachsenden Rohstoffe oft als unerwünschtes Nebenprodukt mitgeführte Wasser kann in einem erfindungsgemäßen Olefin-Konversionsverfahren sinnvoll eingesetzt werden, da so auf einen zusätzlichen Wasserstrom ganz oder teilweise verzichtet werden kann.

Die Erfindung umfasst weiterhin auch eine Anlage zur Herstellung von niedermolekularen Olefinen, insbesondere von Ethylen und Propylen, mit den Merkmalen des Anspruchs 9, die zur Durchführung des erfindungsgemäßen Verfahrens geeignet ist. In wenigstens einem mit Katalysator befüllten Reaktor werden simultan ein wenigstens ein Oxygenat enthaltender Eduktstrom und ein $C_{4+}$-Olefin enthaltender Eduktstrom zu einem niedermolekulare Olefine und Benzinkohlenwasserstoffe umfassenden Produktgemisch umgesetzt, wobei in diesen Reaktor wenigstens eine Zuführungsleitung des Oxygenate enthaltenden Eduktstroms und eine Zuführungsleitung des $C_{4+}$-Olefine enthaltenden Eduktstroms münden und mittels einer Dosiereinrichtung das erfindungsgemäß definierte Verhältnis dieser beiden Eduktströme auf einen Wert zwischen 0,05 und 0,5 einstellbar ist.

Weil das Verhältnis der Oxygenate gegenüber den Olefinen im Einsatz relativ klein ist, befinden sich im Produktgemisch des Reaktors nur noch kleine Restgehalte an Oxygenaten. Deshalb kann die Aufbereitung des Produktgemisches gegenüber dem klassischen MTP®-Verfahren z. T. stark vereinfacht werden

Eine demgemäße Ausgestaltung der Erfindung sieht vor, dass dem Reaktor eine erste Trenneinrichtung zur Auftrennung des erhaltenen Produktgemisches in ein $C_3$-Olefin reiches Gemisch und ein an $C_{4+}$-Benzinkohlenwasserstoffen reiches Gemisch nachgeschaltet ist. Auf diese erste Trenneinrichtung folgt eine zweite Trenneinrichtung zur Auftrennung des an $C_{4+}$-Benzinkohlenwasserstoffen reichen Gemischs in einen im Wesentlichen $C_4$-Fraktionen enthaltenen Strom, einen an $C_{5+}$-Benzinkohlenwasserstoffen reichen Strom und einen vorzugsweise vor allem $C_4$-$C_6$-Olefine enthaltenden Recyclingstrom. Es hat sich als günstig erwiesen, wenn eine Rückführleitung von dieser zweiten Trenneinrichtung zurück zum Reaktor führt. In dieser Rückführleitung ist eine Dosiereinrichtung vorgesehen, mit welcher das Molverhältnis zwischen dem an Aromaten armen Recyclingstrom und dem $C_{4+}$-Olefine enthaltenden Eduktstrom auf einen Wert zwischen 0,1 und 1,5 einstellbar ist. So können höherwertige Benzinkohlenwasserstoffe durch Spaltung doch noch in die Zielprodukte Ethylen und Propylen umgewandelt werden.

Es liegt auch im Rahmen der Erfindung, die zweite Trenneinrichtung in Form einer integrierten Kolonne auszuführen, wobei im oberen Teil ein $C_4$-Schnitt über eine Leitung als Kopfprodukt abgetrennt wird, das Sumpfprodukt über eine Leitung im unteren Teil als Rückfluss oder Einsatz aufgegeben wird und im unteren Teil ein $C_{4-6}$-Schnitt über eine Leitung als Kopfprodukt und ein $C_{5+}$-Schnitt über eine Leitung als Sumpfprodukt entnommen werden.

Der Reaktor kann als typischer Reaktor für eine heterogen-katalysierte Reaktion in einer Gasphase ausgestaltet sein, wie es zum Beispiel ein Rohrbündelreaktor oder auch ein Festbettreaktor ist. Es hat sich als besonders vorteilhaft erwiesen, den Reaktor als Festbettreaktor zu gestalten, wobei der Katalysator in mehreren einzelnen, wenigstens zwei, vorzugsweise vier, Katalysatorfestbetten angeordnet ist. In diesen einzelnen Stufen des Reaktors mündet jeweils eine Leitung, über die Teile des Oxygenate enthaltenden Eduktstroms einströmen. Diese Leitungen werden durch eine Dosiervorrichtung beschickt. Jede Stufe, insbesondere die Stufen 1 und 2 werden auf minimale Verweilzeit/Reservekapazität ausgelegt. Die vierte Stufe ist eine Sicherheitsstufe, die zur Abreaktion der Oxygenate dient. Ebenso werden $C_{6+}$-Olefine ausreagiert, da diese nur teilweise beziehungsweise gar nicht zurückgeführt werden können.

Weiterbildungen, Vorteile und Anwendungsmöglichkeiten der Erfindung ergeben sich auch aus der nachfolgenden Beschreibung von Ausführungsbeispielen und den Zeichnungen. Dabei bilden alle beschriebenen und/oder bildlich dargestellten Merkmale für sich oder in beliebiger Kombination den Gegenstand der Erfindung unabhängig von ihrer Zusammenfassung in den Ansprüchen oder deren Rückbeziehung.

Es zeigen:

Fig. 1    das grundsätzliche Verfahrensschema;

Fig. 2    schematisch eine erfindungsgemäße Anlage mit zwei nachgeschalteten Trenneinrichtungen;

Fig. 3    schematisch eine erfindungsgemäße Anlage mit vier nachgeschalteten Trennungseinrichtungen;

Fig. 4    ein Statusdiagramm für den Umsatz eines $C_4$- bis $C_6$-Olefinschnitts (Reaktordruck: 1,8 bar (a));

Fig. 5    ein Statusdiagramm für den Umsatz eines $C_4$- bis $C_6$-Olefinschnitts (Reaktordruck: 4,5 bar (a));

Fig. 6    schematisch eine Ausführung der Trenneinheit 40 als integrierte Destillationskolonne.

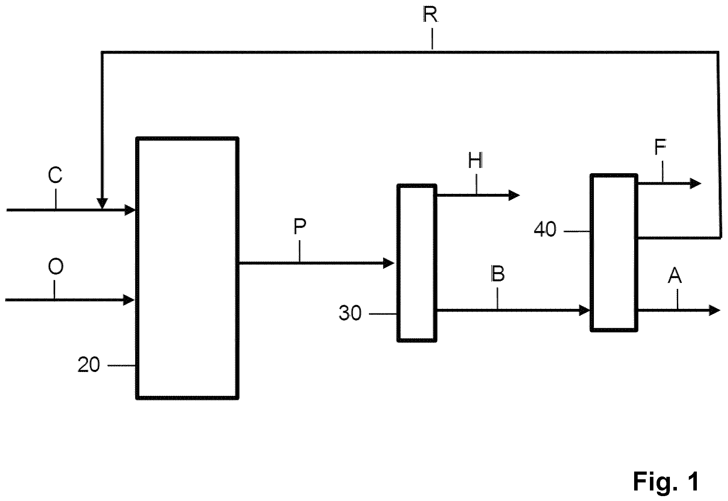

In Fig. 1 ist das grundsätzliche Verfahrensschema dargestellt. In einen Reaktor 20 wird ein wenigstens ein Oxygenat enthaltender Eduktstrom O und ein wenigstens ein $C_{4+}$-Olefin enthaltender Eduktstrom C eingeleitet und dort simultan an einem festen Katalysator, vorzugsweise ein Alumosilikat vom Pentasiltyp ZSM-5, umgesetzt. Der entstehende Produktstrom P wird anschließend in eine erste Trennvorrichtung 30 geleitet. In dieser Trennvorrichtung 30 wird der Strom P in ein an $C_{3-}$-Olefin reiches Gemisch H und ein an $C_{4+}$-Benzinkohlenwasserstoffen reiches Gemisch B aufgetrennt. Der Strom B wird anschließend in eine zweite Trennvorrichtung 40 geleitet und dort in einen im Wesentlichen $C_4$-Fraktionen enthaltenen Strom F, einen an $C_{5+}$-Benzinkohlenwasserstoffen reichen Strom A und einen Recyclingstrom R aufgetrennt. Der Recyclingstrom R, der vorzugsweise vorwiegend $C_4$-$C_6$-Olefine in Gasform enthält, wird anschließend wenigstens teilweise in den Reaktor 20 zurückgeführt.

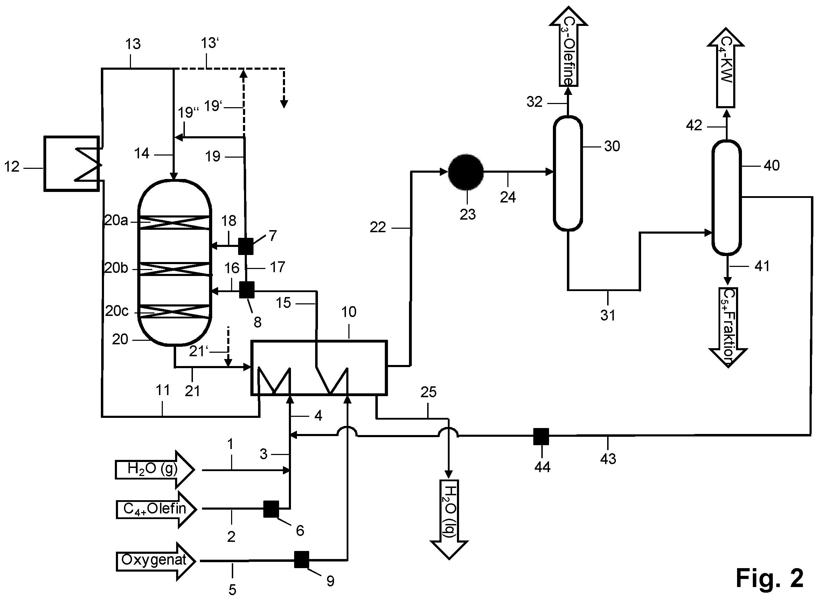

Fig. 2 zeigt die Ausführung einer erfindungsgemäßen Anlage. Dazu wird ein Wasser, vorzugsweise bereits verdampftes Wasser, enthaltender Strom über Leitung 1 einem wenigstens ein $C_{4+}$-Olefin enthaltenden Strom in Leitung 2 zugemischt. Über Leitung 3 und Leitung 4 wird die Mischung dieser Ströme in einen Wärmetauscher 10 eingespeist und dort vorerhitzt. Die Menge an $C_{4+}$-Olefin wird über die Dosiervorrichtung 6 geregelt. Eine ebenfalls vorhandene Dosiereinrichtung für den Wasserdampf ist in der Abbildung nicht dargestellt. In diesen Wärmetauscher 10 wird außerdem ein über Leitung 5 und Dosiereinrichtung 9 zugeführter Oxygenatstrom verdampft und ggf. überhitzt.

Der Olefin / Wasserdampfstrom gelangt über Leitung 11 in eine Unterfeuerung 12 und von dort über die Leitungen 13 und 14 weiter in den Reaktor 20. Die Menge des Stroms wird über Dosiervorrichtungen in den Leitungen 1 und 2 sowie 43 eingestellt.

Über Leitung 15 tritt der vorverdampfte und optimal überhitzte Oxygenatstrom in eine Dosiervorrichtung 8 ein, von der aus er über Leitung 16 und Leitung 18 direkt vor die Katalysator Festbetten 20b und 20c eingespeist wird. Über Leitung 19 ist es zudem möglich, den Oxygenatstrom wenigstens teilweise in die auf die Reaktoreintrittstemperatur vorerhitzte Olefin-Wasserdampf-Mischung zu leiten und gemeinsam mit dieser in den Reaktor 20 vor das Katalysatorbett 20a zu führen.

Die Menge des Oxygenatstroms ist zu der im Olefinstrom enthaltenden Olefinmenge in das erfindungsgemäß definierte Verhältnis zu setzen, weshalb der Oxygenatstrom durch Regelvorrichtungen wie 7 oder 8 eindosiert werden muss. Diese Dosierung, bzw. die Aufteilung auf die einzelnen Katalysatorfestbetten erfolgt so, dass die Temperatur eines aus einem Bett austretenden Reaktionsgasstroms durch die Oxygenatdosierung vor dem Bett auf den gewünschten Wert eingestellt wird. Das letzte Katalysatorbett 20c wird mit einer geringen Belastung an Oxygenaten ausgelegt, damit der Oxygenatumsatz dort nahezu quantitativ wird. Entsprechend dem $C_{4+}$-Olefingehalt und dem Anteil des zugeführten Oxygenats stellt sich über ein Bett jeweils ein Temperaturabfall oderanstieg über wenige Grad Celsius ein, wodurch über den gesamten Reaktor 20 eine Temperaturdifferenz von ca. -5 bis + 13°C entsteht.

Grundsätzlich wäre es auch denkbar, den wasserhaltigen Strom wenigstens teilweise über Leitung 1 direkt in den Reaktor 20 einzuspeisen, was eine weitere Dosiervorrichtung für die zugeführte Wassermenge erfordern würde. Auch eine wenigstens teilweise Zumischung des Wasserstroms in den Oxygenatstrom ist grundsätzlich denkbar.

Ebenso ist denkbar, einen Teil des $C_{4+}$ Olefin Eduktstroms von Leitung 2 oder einen Teil des olefinhaltigen Recyclingstroms von Leitung 43 zur Leitung 5 oder direkt zu einer oder mehreren der Leitungen 18, 16, 16' zu führen und damit dem Reaktor 20 zuzuführen. Auch hierfür wäre mindestens eine weitere Dosiereinrichtung erforderlich.

Zudem können über Leitung 13' und 19' Teile der Eduktströme in einen Parallelreaktor eingeführt werden, über Leitung 19" können Teile des Stroms aus Leitung 19 in Leitung 14 gespeist werden. Auch hier ist eine Dosiereinrichtung möglich. Parallelreaktoren dienen zum einen dazu, das Alterungsverhalten des Katalysators abzupuffern, indem Reaktoren zu verschiedenen Zeitpunkten regeneriert werden. Ein Parallelreaktor kann entweder genauso wie der Reaktor 20 betrieben werden oder auch eine andere Konfiguration, zum Beispiel eine andere Menge von Katalysatorfestbetten aufweisen. Eine solche Parallelverschaltung von Reaktoren ermöglicht eine größere Flexibilität der Anlage hinsichtlich der umgesetzten Massenströme.

Über Leitung 21' kann ein in einem eventuell vorhandenen Parallelreaktor entstandener Produktstrom dem Produktstrom aus Reaktor 20 in Leitung 21 zugemischt werden. Die Summe der Produktströme tritt über Leitung 21 in den Wärmetauscher 10 ein und wird damit gleichzeitig dazu genutzt, die Eduktströme zu erwärmen.

In der Wärmerückgewinnungsvorrichtung 10 - ein ebenfalls vorhandener integrierter Schlusskühler ist in der Abbildung nicht dargestellt - findet zudem bereits eine Abtrennung der wässrigen von der organischen Phase statt. Die Gasphase wird über Leitung 22 einem Verdichter 23 zugeführt, von dem aus sie über Leitung 24 in eine erste Trennvorrichtung 30 gelangt. Das auf etwa 25 bar komprimierte Produktgemisch wird in der Trennvorrichtung 30 zunächst in die $C_{3-}$-Olefine und eine Fraktion der längerkettigen Olefine aufgetrennt. Während die $C_{3-}$-Olefine über Leitung 32 ausgeschleust werden, gelangt das Sumpfprodukt mit den längerkettigen Olefinen über Leitung 31 in die zweite Trennvorrichtung 40, welche ebenso wie die Trennvorrichtung 30 vorzugsweise als Destillationskolonne ausgestaltet ist. In dieser zweiten Trennvorrichtung 40 werden die $C_4$-Kohlenwasserstoffe als Kopf- und die mindestens fünf Kohlenstoffatome enthaltenden Kohlenwasserstoffe ($C_{5+}$-KW) als Sumpfprodukte abgetrennt. Aus der Trennvorrichtung 40 wird zudem ein Rückführstrom mit einem möglichst großen $C_4$- bis $C_6$-Olefinrestgehalt über Leitung 43 in Leitung 3 zurückgeführt. Die Menge dieses Stroms wird über die Regeleinrichtung 44 bestimmt. Vorzugsweise wird der Rückführstrom in Gasform zum Reaktor zurückgeführt. Dazu wird die Trenneinrichtung 40 bei einem Druck von ~4 bar oberhalb des Reaktordrucks betrieben und über Leitung 43 ein Gas-Teilstrom an einer geeigneten Position aus der Trenneinrichtung entnommen.

Wird die Trenneinheit 40 als integrierte Destillationskolonne nach Fig. 6 ausgeführt, wird über Leitung 42 eine $C_4$ Fraktion entnommen, wobei die Menge durch den Aufkocher am oberen Teil der Kolonne bestimmt wird. Die restliche Menge an $C_4$ gelangt mit $C_5$ Kohlenwasserstoffen über Leitung 46 in den unteren Teil der Kolonne und wird mit einer vorteilhaften Menge $C_{5-6}$ Kohlenwasserstoffen über Leitung 43 zum Reaktor geführt. Über die Regelung des Aufkochers am unteren Teil der Kolonne wird bestimmt, welcher Teil der $C_{5+}$ Kohlenwasserstoffe zum Kopf der Kolonne geführt bzw. als Sumpfprodukt gewonnen wird. Die aromatenreiche Fraktion $C_{6+}$ wird vorwiegend über Leitung 31 als Produkt aus dem Prozess herausgeführt.

In der Einheit zur Wärmerückgewinnung 10 wird aus den Reaktionsgasen Wasserdampf kondensiert und abgetrennt. Aus dem wässrigen Kondensat kann in einem nicht dargestellten Stripper eine oxygenathaltige Fraktion zurückgewonnen und in den Reaktor 20 rückgeführt werden.

Die in Fig. 2 dargestellte Minimalkonfiguration kann vor allem in Fällen mit einem im Vergleich geringen Oxygenateinsatz Anwendung finden. Ein das Propylenprodukt kompromittierender Restgehalt an leichtflüchtigen Reaktionsprodukten, z.B. Dimethylether (DME) kann ohne besonderen apparatemäßigen Aufwand spätestens im nicht dargestellten $C_3$-Splitter mit dem Sumpfprodukt Propan abgetrennt werden.

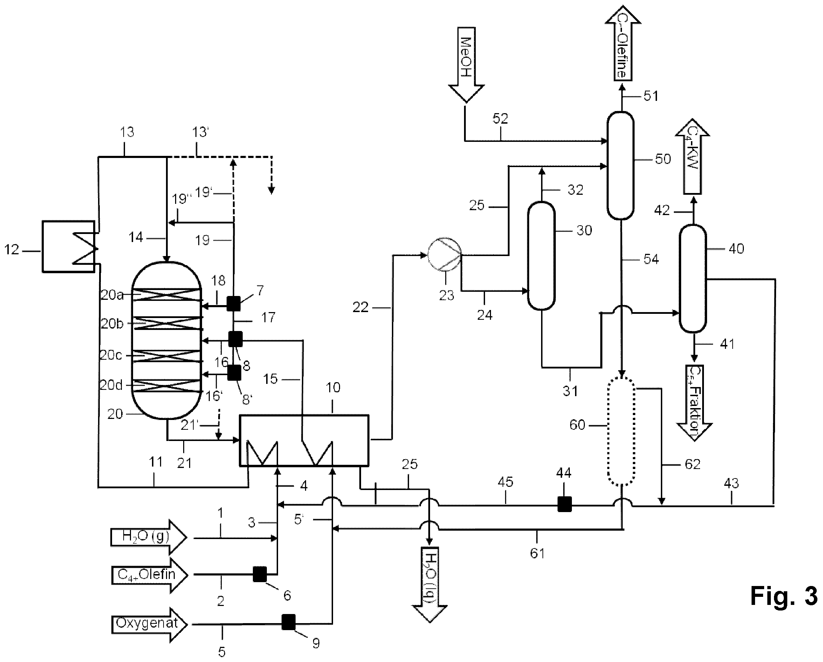

Sollte dies nicht möglich sein, weil besondere Anforderungen an das Propanprodukt gestellt werden oder weil mit sehr hohem Oxygenateinsatz eine hohe Propylenausbeute erzeugt werden soll, dann kann eine aufwendigere Konfiguration zur Anwendung kommen, die in Fig. 3 dargestellt ist.

Der Aufbau der Reaktoreinheit ähnelt dabei derjenigen, die bereits in Fig. 2 beschrieben ist. Allerdings ist der Reaktor als vierstufiger Reaktor vorgesehen, weshalb über Leitung 16' und Dosiervorrichtung 8' ein weiterer Oxygenatteilstrom in den Reaktor 20 eingespeist wird.

Nach der Kompression auf etwa 25 bar in dem Kompressor 23 werden die Produktsströme über die Leitungen 24 und 25 zunächst einer Trennvorrichtung bestehend aus den Kolonnen 30 und 50 zugeführt. Diese Trenneinrichtung ist beschrieben in der DE 102008058931 A1 und trennt mit Hilfe eines Waschmittels - hier Methanol - das Produktgemisch in eine oxygenatfreie $C_{3-}$ Fraktion (Leitung 51), einen $C_{4+}$ Strom (Leitung 31) sowie das vorwiegend mit DME/ $C_{4-5}$ Kohlenwasserstoffen beladene Waschmittel (Leitung 54). Der flüssige Produktstrom wird über Leitung 24 zur Trennvorrichtung 30 geführt und in eine kurzkettige ($C_{3-}$-Olefine) und eine langkettige ($C_{4+}$-Olefine) Fraktion aufgeteilt. Die kurzkettige Fraktion wird über Leitung 32 in Leitung 25 zugemischt und der kombinierte - die gesamte $C_{3-}$-Olefinausbeute enthaltende - Strom der Trenneinrichtung 50 zugeführt. , Dort werden restliche Mengen an $C_{4+}$ Kohlenwasserstoffen aus dem $C_{3-}$ Kopfprodukt abgetrennt und gleichzeitig mit Hilfe eines Oxygenat-Waschmittels vorzugsweise Methanol, leichtflüchtigen Oxygenate, vor allem DME, quantitativ aus dem $C_{3-}$ Produkt entfernt... Die $C_{3-}$-Olefine werden dann über Leitung 51 ausgeführt.

Im weiteren Fortgang wird - über DE 102008058931 A1 hinausgehend - der Methanol/Oxygenat/Olefinstrom über Leitung 61 entweder direkt in Leitung 5 dem Oxygenateinsatz zugemischt und über Leitung 5' in die Vorrichtung zur Wärmerückgewinnung 10 eingespeist. Oder es wird eine zusätzliche Trennvorrichtung 60 geschaltet, durch die die

enthaltenen $C_{4-5}$ Olefine abgetrennt und über Leitung 62 abgezogen werden können. Leitung 62 mündet in Leitung 43, wodurch der vereinte Olefinstrom über Leitung 44 in Leitung 3 rückgeführt werden kann. Das Methanol-Sumpfprodukt der Kolonne 60 wird über Leitung 61 dem Oxygenat-Einsatz beigemischt. Die Trennung von $C_{4-5}$ Kohlenwasserstoffen von Methanol hat den Effekt, daß die Kohlenwasserstoffe bei hoher Temperatur dem Reaktor zugeführt werden können und demnach bei sehr niedrigen Oxygenat / Olefin Verhältnissen die Energiebilanz verbessern. Umgekehrt kann bei einem relativ hohen Oxygenateinsatz auf eine weitere Trennvorrichtung 60 verzichtet werden, weil durch die "Verdünnung" des Oxygenates durch Olefine ein stärkerer Kühleffekt vor der Aufgabe auf das Katalysatorbett erzielt wird, was bei hohen Oxygenateinsätzen erwünscht ist.

Die längerkettigen Kohlenwasserstoffe werden aus der Trenneinrichtung 30 über Leitung 31 in eine Trennvorrichtung 40 eingebracht, in der die $C_4$- von den $C_{5+}$-Kohlenwasserstoffen getrennt werden. Aus dem Kopf der Destillationskolonne 40 werden über Leitung 42 $C_4$ Kohlenwasserstoffe ausgeschleust, während die $C_{5+}$ Kohlenwasserstoffe die Kolonne über Leitung 41 aus dem Sumpf der Kolonne 40 verlassen. Ferner kann über Leitung 43 ein Recyclingstrom aus der Kolonne 40 über Leitung 44 in Leitung 3 rückgeführt werden. Die Position der Leitung 43 in der Kolonne 40 wird so bestimmt, dass entsprechend der Zusammensetzung des Einsatzes in der Leitung 31 ein maximaler $C_4$-$C_6$ Anteil des Recyclingstroms in Leitung 43 eingestellt werden kann. Vorzugsweise wird die Kolonne bei einem Druck von > 4 bar oberhalb des Reaktordrucks betrieben, damit der Recyclingstrom ohne Kompression in Gasform zum Reaktor zurückgeführt werden kann.

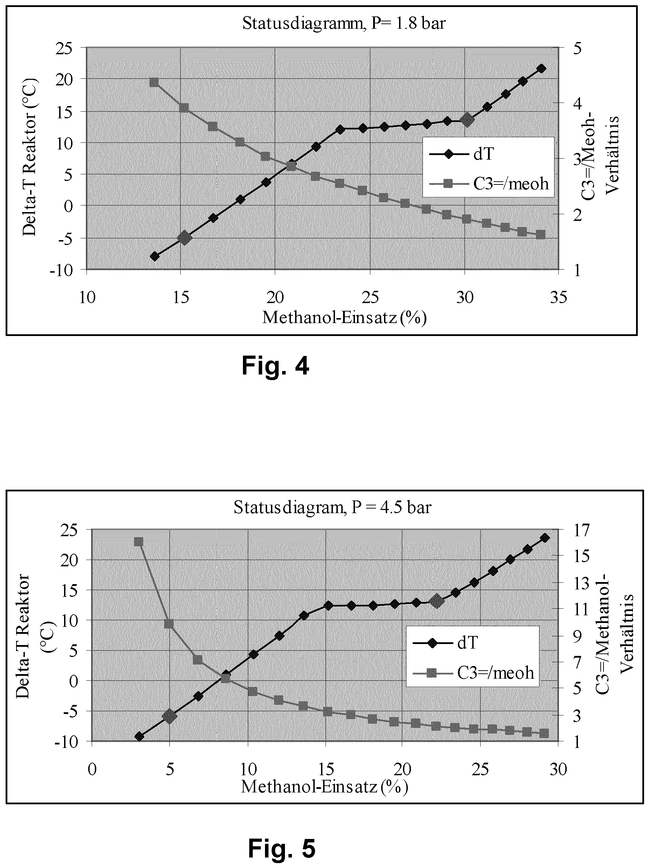

In Fig. 4 sind wesentliche der genannten Zusammenhänge für einen typischen $C_4$-$C_6$-Olefinschnitt aus einer Cracker-Anlage veranschaulicht. Bei einem Reaktordruck von 1.8 bar (a) stellt sich bei einem Methanol-Einsatz von 15 - 30 % definiert nach dem erfindungsgemäßen Mengenverhältnis V ein Temperaturprofil über den Gesamtreaktor von -5 bis +13°C ein. Der Temperatur-Haltebereich wird dabei durch unterschiedliche Vorwärmtemperaturen des Methanols eingestellt. Das Propylen:Methanol-Verhältnis beträgt 3.6 - 1.5. Bei einem Olefin-Gesamtumsatz von 80 - 75 % liegt die erzielbare Selektivität bezogen auf die Gesamtmenge Ethylen und Propylen bei 59.5 - 57%.

Wie beim MTP®-Prozess begünstigt ein möglichst niedriger Druck - d. h. ca. 1.7 - 1.9 bar (a) im Reaktoreintritt - die Propylen- und Ethylenausbeuten. Weil aber $C_{4+}$-Olefine z.T. unerwünschte und niedriger bewertete Produkte darstellen, kann der Eintrittsdruck in einem erfindungsgemäßen Reaktor auf 3 - 5 bar angehoben werden, ohne dass dadurch die Wirtschaftlichkeit des Verfahrens zunichte gemacht wird. Der hohe Druck ermöglicht eine kompakte Ausführung der Anlage und geringere Investitions- und Betriebsmittelkosten. Auf eine maximale Propylenausbeute wird unter diesen Randbedingungen verzichtet. Je höher der Druck, desto niedriger die Propylenausbeute und umso weniger Methanol (Oxygenat) wird zur Einstellung der Temperatur benötigt.

Fig. 5 zeigt, wie sich bei einem Reaktordruck von 4.5 bar (a) bei einem Methanol-Einsatz (= V * 100) von 5 - 23 % ein Temperaturprofil von -5 bis +13°C einstellt. Das Propylen:Methanol-Verhältnis beträgt 10:2. Bei einem Olefin-Gesamtumsatz von 85 - 80 % liegt die erzielbare Selektivität bezogen auf die Gesamtmenge Ethylen und Propylen bei 49-48%.

Beispiel 1

In Beispiel 1 wird angenommen, dass aus einem für eine Cracker-Anlage typischen $C_{4+}$-Olefinschnitt von 62 t/h mit 67 Massen-% Olefine, davon 53 % $C_4$-Olefine wirtschaftlich sinnvoll eine möglichst große Menge an Propylen und Ethylen erzeugt werden soll. Dazu wird eine Olefin-Methanol-Konversionsanlage, wie sie in Fig. 3 dargestellt ist, konfiguriert, die bei 1.8 bar Reaktordruck und einer relativ hohen Rückführungsrate (1.38) und Methanol-Einsatz (24 %) arbeiten soll. Die Reaktor-Zieltemperatur beträgt ~497 °C. Im Falle von nur drei Trennvorrichtungen 30, 40 und 50 ist mit einer Temperaturerhöhung von 7,5 °C zu rechnen. Die Trenneinheit 40 ist als integrierte Kolonne nach Fig. 6 ausgeführt.

In Tabelle 1 ist eine Massenbilanz, in Tabelle 2 sind die Einsatz/Produktmengen und wichtige Kennzahlen eines solchen Prozesses zusammengestellt.

Für den Reaktor wird eine Gesamthöhe von ~9 m und ein Durchmesser von 5,5 m angenommen.

Tab. 1 Massenbilanz eines Verfahrens nach Beispiel 1

| Leitung Nr. | 2 | 5 | 22 | 51 | 42 | 41 | 43 |
|---|---|---|---|---|---|---|---|
| Massenfluss [kg/h] | | | | | | | |
| MEOH | 0.00 | 30000.00 | 397.05 | 0.03 | 0.03 | 0.00 | 32.76 |
| DME | 0.00 | 0.00 | 31.15 | 0.00 | 0.01 | 0.00 | 0.12 |
| H2O | 0.00 | 0.00 | 7557.60 | 0.00 | 0.00 | 0.00 | 309.72 |
| C2H4 | 0.00 | 0.00 | 5377.64 | 5167.57 | 0.00 | 0.00 | 14.79 |

(fortgesetzt)

| Leitung Nr. | 2 | 5 | 22 | 51 | 42 | 41 | 43 |
|---|---|---|---|---|---|---|---|
| C3H6 | 0.00 | 0.00 | 28059.67 | 27348.31 | 114.82 | 0.00 | 161.19 |
| C4= | 32573.19 | 0.00 | 19543.05 | 0.00 | 4584.01 | 0.92 | 13874.14 |
| C5= | 3886.99 | 0.00 | 10551.97 | 0.00 | 10.38 | 3027.11 | 7389.67 |
| C6-C8= | 5056.42 | 0.00 | 3403.66 | 0.00 | 0.00 | 2320.84 | 1079.64 |
| CH4 | 0.00 | 0.00 | 204.66 | 198.50 | 0.00 | 0.00 | 0.43 |
| C2 | 0.00 | 0.00 | 41.09 | 40.69 | 0.00 | 0.00 | 0.03 |
| C3 | 84.76 | 0.00 | 1559.61 | 1483.84 | 26.14 | 0.00 | 35.34 |
| C4 | 12755.22 | 0.00 | 67131.53 | 0.00 | 15199.85 | 4.17 | 47307.18 |
| C5 | 2745.57 | 0.00 | 15839.87 | 0.00 | 62.77 | 3964.12 | 11600.37 |
| C6-C8 | 4728.04 | 0.00 | 9380.06 | 0.00 | 0.00 | 6391.67 | 2984.28 |
| Aromaten | 163.61 | 0.00 | 5425.31 | 0.00 | 0.00 | 4981.30 | 449.28 |
| H2 | 0.00 | 0.00 | 23.94 | 23.92 | 0.00 | 0.00 | 0.00 |
| CO | 0.00 | 0.00 | 214.74 | 214.57 | 0.00 | 0.00 | 0.01 |
| Inertgase | 0.00 | 0.00 | 3.25 | 3.20 | 0.00 | 0.00 | 0.00 |
| Oxygenate | 0.00 | 0.00 | 217.29 | 0.00 | 0.00 | 42.33 | 160.77 |
| | | | | | | | |
| Gesamtfluss [kmol/h] | 1012.50 | 936.27 | 3435.75 | 901.08 | 347.55 | 245.44 | 1405.48 |
| Gesamtfluss [kg/h] | 61993.80 | 30000.00 | 174963.00 | 34480.62 | 19998.00 | 20732.46 | 85399.70 |
| Gesamtfluss [m^3/h] | 108.90 | 38.51 | 88989.03 | 777.43 | 43.65 | 29.82 | 2759.70 |
| Temperatur [°C] | 53.38 | 30.00 | 50.00 | 45.08 | 98.95 | 38.09 | 105.29 |
| Druck [bar] | 18.00 | 1.00 | 1.02 | 23.00 | 17.00 | 13.18 | 12.80 |
| Dampffraktion | 0.00 | 0.00 | 1.00 | 1.00 | 0.00 | 0.00 | 1.00 |
| Flüssige Fraktion | 1.00 | 1.00 | 0.00 | 0.00 | 1.00 | 1.00 | 0.00 |
| Enthalpie [J/kg] | -1.20E+06 | -7.45E+06 | -1.65E+06 | 4.88E+05 | -1.91 E+06 | -1.37E+06 | -1.60E+06 |
| Dichte [kg/m^3] | 569.27 | 779.08 | 1.97 | 44.35 | 458.18 | 695.28 | 30.95 |
| Mittlere Molekülmasse | 61.23 | 32.04 | 50.92 | 38.27 | 57.54 | 84.47 | 60.76 |

Tab. 2 Einsatz- und Produktmengen für die Beispiele 1, 2 und 3

| | Einheit | | Beispiel 1 | Beispiel 2 | Beispiel 3 |
|---|---|---|---|---|---|
| | | | | | |
| Einsatz | t/h | | 62.0 | 62.0 | 40.0 |
| Einsatz- Typ | | | $C_4$-$C_6$ | $C_4$-$C_6$ | $C_4$ |
| Olefingehalt | % | | 67.0 | 67.0 | 50.0 |
| Oxygenat-Typ | | | Methanol | Methanol | $C_1$-$C_5$ Alkohole |
| Oxygenat-Menge | t/h | | 30.0 | 9.0 | 9.0 |
| | | | | | |
| Propylen | t/h | | 27.4 | 17.7 | 10.1 |

(fortgesetzt)

| | Einheit | | Beispiel 1 | Beispiel 2 | Beispiel 3 |
|---|---|---|---|---|---|
| Ethylen | t/h | | 5.2 | 3.6 | 2.1 |
| | | | | | |
| C4 | t/h | | 20.0 | 19.0 | 16.0 |
| C4+ oder C5+ | t/h | | 20.7 | 21.9 | 15.0 |
| | | | | | |
| Oxygenat(Methanol)-Anteil | % | | 24.0 | 8.7 | 17.8 |
| Propylen/Oxygenat-Verhältnis | | | 2.5 | 5.4 | 2.9 |
| Propylen-Selektivität | % | | 59.4 | 46.8 | 51.9 |
| Olefin-Umsatz | % | | 76.1 | 81.2 | 70.8 |

**Beispiel 2**

In Beispiel 2 wird eine Umkonstruktion einer bestehenden Cracker-Anlage in eine erfindungsgemäße Anlage (Revamp-Situation) angenommen. Aus demselben $C_{4+}$-Olefinschnitt wie in Beispiel 1 sollen mit minimalen Investitionskosten zusätzliche Mengen an Propylen und Ethylen erzeugt werden. Gleichzeitig sollen der Mengenstrom und Olefingehalt der $C_{4+}$-Ströme deutlich reduziert werden, um die Kapazität der nachgeschalteten Verarbeitungsanlagen zu schonen. Dazu wird eine Olefin/Methanol-Konversionsanlage, wie in Fig. 2 dargestellt, konfiguriert, die bei 4.5 bar Reaktordruck und mittlerer Rückführungsrate (1.0) und nur 9% Methanol-Einsatz arbeiten soll. Die Reaktor-Zieltemperatur beträgt ~497 °C. Auch hier wird die Trenneinheit 40 als integrierte Kolonne nach Fig. 6 ausgeführt.

In Tabelle 2 sind die Einsatz/Produktmengen und wichtige Kennzahlen zusammengestellt. Für den Reaktor wird eine Gesamthöhe von ~7 m und ein Durchmesser von 3,4 m angenommen. Im Vergleich zum Beispiel 1 verringern sich die Dimensionen der nachgeschalteten Anlagen bis zum Kompressor entsprechend.

**Beispiel 3**

Auch in diesem Beispiel wird eine Revamp-Situation in einer Cracker-Anlage angenommen. Aus einem $C_{4+}$-Olefinschnitt (40 t/h, 50% % Olefinanteil) sollen mit minimalen Investitionskosten zusätzliche Mengen an Propylen und Ethylen erzeugt werden. In diesem Fall stehen neben Methanol 3 t/h Alkoholmischung mit $C_2$-$C_5$ Alkoholen, die als Nebenprodukt in einer Bioethanol-Anlage anfallen, zur Verfügung. Dazu wird eine Olefin/Methanol-Konversionsanlage, wie in Fig. 2, dargestellt konfiguriert, die bei 4.5 bar Reaktordruck und mittleren Rückführungsrate (1.0) arbeiten soll. Der Oxygenat-Anteil beträgt 29%. Die Reaktor-Zieltemperatur beträgt ~484 °C; das Temperaturprofil über den Reaktor beträgt -4.5°C. Der leichte Temperaturabfall in diesem Fall ist vor allem dadurch bedingt, dass die Alkoholmischung auch Ethanol enthält, welches sich in einer hoch endothermen Reaktion hauptsächlich zu Ethylen umsetzt. In Tabelle 2 sind die Einsatz/Produktmengen und wichtige Kennzahlen zusammengestellt

Für den Reaktor wird eine Gesamthöhe von ~7 m und ein Durchmesser von 2,8 m angenommen.

**Beispiel 4**

In Tabelle 3 sind Kenndaten für verschiedene erfindungsgemäße Olefin-Konversionsanlagen zusammengestellt und mit dem Propylur-Prozess verglichen. Einsatz ist im Beispiel derselbe $C_{4+}$ Schnitt wie in den Beispielen 1 und 2. Bezüglich der Ausbeuten an Propylen und Ethylen und des Betriebsmittelverbrauches sind deutliche, z. T drastische Vorteile des erfindungsgemäßen Verfahrens gegenüber dem Propylur-Verfahren festzustellen. Dasselbe würde auch für das OCP-Verfahren gelten. In diesen Beispielen ist die Trenneinheit 40 als einfache Kolonne mit gasförmigem Seitenabzug ausgeführt.

Tabelle 3 Vergleich des erfindungsgemäßen Prozesses mit dem Propylur-Verfahren

| | Recyclingstrom [t/h] | Dampf [t/h] | Alkohol [t/h] | Ausbeute [1] (Ethylen+ Propylen) [t/h] | Ausbeute [1] (Ethylen+ Propylen) [Mol-%] | Betriebsmittel-Verbräuche | | |
| | | | | | | Dampf[2] | Ofen[3] | Kompressor |
|---|---|---|---|---|---|---|---|---|
| Propylur | 31,0 | 141,0 | | 19,8 | 47,2 | 146,0 | 17,0 | 3,8 |
| Olefin-Konversion 500 °C, 3,5 bar | 88,0 | 30,0 | 23,0 | 27,6 | 53,5 | 50,5 | 20,5 | 3,5 |
| Olefin-Konversion 500 °C, 4,5 bar | 90,0 | 30,0 | 13,0 | 23,2 | 49,2 | 51,0 | 17,0 | 2,1 |
| Olefin-Konversion mit Bioethanol[4] 485 °C, 4,5 bar[4] | 88,0 | 30,0 | 20,0 | 27,4 | 52,8 | 51,0 | 20,0 | 2,6 |
| [1] berechnet auf Alkohole und Olefine<br>[2] inklusive Prozessdampferzeugung<br>[3] Antriebsleistung<br>[4] Alkoholeinsatz: 50% Bio-Rohethanol (inkl. $C_3$- bis $C_6$ -Alkohole), 50 % Methanol; Ethanol liefert vorwiegend Ethylen. Ausbeuten sind Schätzwerte | | | | | | | | |

EP 2 688 859 B1

**Beispiel 5**

In einer Fischer-Tropsch-Anlage (F-T-Anlage) entstehen Olefine und Alkohole als Nebenprodukte. Diese werden hydriert bzw. für Unterfeuerungszwecke verwendet. In einer mit der F-T-Anlage integrierten erfindungsgemäßen OlefinKonversionsanlage können die olefin- und alkoholhaltigen Ströme zur Propylenproduktion eingesetzt werden. Die Ausbeuten sind in Tabelle 4 dargestellt.

Tabelle 4 Vergleich des erfindungsgemäßen Prozesses im Verbund mit einer F-T-Anlage zu einer alleinstehenden F-T-Anlage auf der Basis von 80000 barrel / day

|  | Olefinhaltige Kohlenwasserstoffe [t/h] | F-T-Alkohole [t/h] (2) | Methanol [t/h] | Ausbeute [1] ($C_3$) [t/h] |
|---|---|---|---|---|
| Standard F-T | 35,3 | - | - | 2,5 |
| F-T + Olefin-Konversion | 35,3 | 7,3 | 10,0 | 10,0 |
| [1] $C_3$-Ausbeute umgerechnet in $C_3$ (Propylen) (99,9%) [2] Alkohol-Gehalt 35 % | | | | |

**Bezugszeichenliste:**

| | |
|---|---|
| 1-5 | Leitungen |
| 6-9 | Dosiervorrichtungen |
| 10 | Wärmerückgewinnungsvorrichtung |
| 11 | Leitung |
| 12 | Unterfeuerung |
| 13-19 | Leitungen |
| 20 | Reaktor |
| 20a-d | Katalysatorfestbetten |
| 21-22 | Leitungen |
| 23 | Verdichter |
| 24-25 | Leitungen |
| 30 | Trenneinrichtung |
| 31-32 | Leitung |
| 40 | Trenneinrichtung |
| 41-43 | Leitungen |
| 44 | Dosiervorrichtung |
| 45 | Leitung |
| 46 | Leitung |
| 50 | Trenneinrichtung |
| 51, 52, 54 | Leitungen |
| 60 | Trenneinrichtung |
| 61 | Leitung |

| | |
|---|---|
| O | Oxygenathaltiger Eduktstrom |
| C | $C_{4+}$ -Olefinhaltiger Strom |
| P | Produktstrom |
| H | $C_{3-}$ -Olefinstrom |
| B | $C_{4+}$ -Olefinhaltiger Strom |
| F | $C_4$ -Olefinhaltiger Strom |
| A | $C_{5+}$ -Olefinhaltiger Strom |
| R | $C_{4-6}$ -Olefinhaltiger Recyclingstrom |

**Patentansprüche**

1. Verfahren zur Herstellung von Ethylen und Propylen, wobei ein wenigstens ein Oxygenat enthaltender Eduktstrom (O) und ein wenigstens ein $C_{4+}$-Olefin enthaltender Eduktstrom (C) simultan in wenigstens einem gleichen Reaktor an einem gleichen Katalysator zu einem niedermolekulare Olefine und Benzinkohlenwasserstoffe umfassenden Produktgemisch (P) umgesetzt werden, **dadurch gekennzeichnet, dass** das Verhältnis (V) von Oxygenaten im Eduktstrom (O) zu $C_{4+}$-Olefinen im Eduktstrom (C) 0,05 bis 0,5 beträgt, wobei sich das Verhältnis (V) nach der Formel

$$V = \frac{\sum_{j} k_{Oxygenat-j} * n_{Oxygenat-j}}{\sum_{i} k_{Olefin-i} * n_{Olefin-i} + \sum_{j} k_{Oxygenat-j} * n_{Oxygenat-j}}$$

berechnet, mit:

$k_{Oxygenat-j}$ : Kohlenstoff-Zahl des Oxygenats j
$n_{Oxygenat-j}$ : Molenstrom des Oxygenats j
$k_{Olefin-i}$ : Kohlenstoff-Zahl des Olefins i
$n_{Olefin-i}$ : Molenstrom des Olefins i

und dass das Produktgemisch (P) in einer ersten Trenneinrichtung in ein $C_3$-olefinreiches Gemisch (H) und einen $C_{4+}$-olefinhaltigen Strom (B) aufgetrennt wird, dass in einer zweiten Trenneinrichtung der $C_{4+}$-olefinhaltige Strom (B) in einen im Wesentlichen $C_4$-Fraktionen enthaltenden Strom (F), einen an $C_{5+}$-Benzinkohlenwasserstoffen reichen Strom (A) und einen vor allem $C_4$-$C_6$-Olefine enthaltenden Recyclingstrom (R) aufgetrennt wird und dass der Recyclingstrom (R) wenigstens teilweise zu dem wenigstens einen Reaktor zurückgeführt wird, wobei das Molverhältnis zwischen dem Recyclingstrom (R) und dem $C_{4+}$-Olefin enthaltenden Eduktstrom (C) zwischen 0,1 und 1,5 liegt, und

dass die zweite Trenneinrichtung bei einem Druck von 4-15 bar betrieben wird und der Recyclingstrom (R), in Gasform entnommen, und unmittelbar in eine in den Reaktor mündende Leitung zurückgeführt wird.

2. Verfahren nach Anspruch 1, **dadurch gekennzeichnet, dass** der Recyclingstrom (R) als Seitenabzug entnommen wird.

3. Verfahren nach einem der vorherigen Ansprüche, **dadurch gekennzeichnet, dass** der Oxygenate enthaltende Eduktstrom (O) in mehrere Teilströme geteilt und jeder Teilstrom auf eines von wenigstens zwei, vorzugsweise vier Katalysatorbetten in dem Reaktor geleitet wird.

4. Verfahren nach einem der vorherigen Ansprüche, **dadurch gekennzeichnet, dass** als Katalysator ein formselektives Zeolithmaterial, vorzugsweise ein Alumosilikat vom Pentasiltyp ZSM-5, verwendet wird.

5. Verfahren nach einem der vorherigen Ansprüche, **dadurch gekennzeichnet, dass** der Oxygenate enthaltende Eduktstrom (O) wenigstens einen Alkohol, vorzugsweise Methanol, enthält.

6. Verfahren nach einem der vorherigen Ansprüche, **dadurch gekennzeichnet, dass** der Druck am Eintritt des Reaktors zwischen 1,5 und 10 bar, vorzugsweise zwischen 1,8 und 5 bar, liegt.

7. Verfahren nach einem der vorherigen Ansprüche, **dadurch gek nnzeichnet,** dass die Temperatur am Austritt des Reaktors zwischen 460 und 560 °C, vorzugsweise zwischen 480 und 510 °C liegt.

8. Verfahren nach einem der vorherigen Ansprüche, **dadurch gekennzeichnet, dass** der Oxygenate enthaltende Eduktstrom (O) wenigstens ein Oxygenat enthält, das bei der Produktion von Ethanol durch Fermentation und/oder bei einer Fischer-Tropsch-Synthese als Nebenprodukt angefallen ist, und/oder dass der $C_{4+}$-Olefine enthaltende Eduktstrom (C) wenigstens ein $C_4$ bis $C_{10}$-Olefin enthält, das bei einer Fischer-Tropsch-Synthese als Primärprodukt angefallen ist.

9. Anlage zur Herstellung von Ethylen und Propylen, insbesondere zur Durchführung eines Verfahrens nach einem der vorhergehenden Ansprüche, mit wenigstens einem katalytischen Reaktor (20) zur simultanen Umsetzung eines wenigstens ein Oxygenat enthaltenden Eduktstroms (O) und eines wenigstens ein $C_{4+}$-Olefin enthaltenden Eduktstroms (C), in den wenigstens eine Zuführungsleitung (14) des Eduktstroms (O) und eine Zuführungsleitung (16) des Eduktstroms (C) münden, **gekennzeichnet durch** wenigstens eine Dosiereinrichtung (6, 7, 8, 9, 8'), mit welcher das Verhältnis (V) von Oxygenaten zu $C_{4+}$-Olefinen auf einen Wert zwischen 0,05 und 0,5 einstellbar ist, wobei sich das Verhältnis (V) nach der Formel

$$V = \frac{\sum\limits_{j} k_{Oxygenat-j} * n_{Oxygenat-j}}{\sum\limits_{i} k_{Olefin-i} * n_{Olefin-i} + \sum\limits_{j} k_{Oxygenat-j} * n_{Oxygenat-j}}$$

berechnet, mit:

$k_{Oxygenat-j}$ : Kohlenstoff-Zahl des Oxygenats j
$n_{Oxygenat-j}$ : Molenstrom des Oxygenats j
$k_{Olefin-i}$ : Kohlenstoff-Zahl des Olefins i
$n_{Olefin-i}$ : Molenstrom des Olefins i

eine erste Trenneinrichtung (30) zur Auftrennung des in dem Reaktor (20) erhaltenen Reaktionsgemischs in ein $C_{3-}$-olefinreiches Gemisch und ein $C_{4+}$-olefinhaltiges Gemisch,
eine mit einem Druck von 4-15 bar betriebene zweite Trenneinrichtung (40) zur Auftrennung des $C_{4+}$-olefinhaltigen Gemischs in einen im Wesentlichen $C_4$-Fraktionen enthaltenden Strom, einen an $C_{5+}$-Benzinkohlenwasserstoffen reichen Strom und einen in Gasform entnommenen und vor allem $C_4$-$C_6$-Olefine enthaltenden Recyclingstrom (R), eine Rückführleitung (43) für den Recyclingstrom (R), die von der zweiten Trenneinrichtung (40) zu dem Reaktor (20) führt, und
eine Dosiereinrichtung (44), mit welcher das Molverhältnis zwischen dem Recyclingstrom (R) und dem Eduktstrom (C) auf einen Wert zwischen 0,1 und 1,5 einstellbar ist.

10. Anlage nach Anspruch 9 , **dadurch gekennzeichnet, dass** die zweite Trenneinrichtung (40) in Form einer integrierten Kolonne ausgeführt ist, wobei im oberen Teil ein $C_4$-Schnitt über eine Leitung (42) als Kopfprodukt abgetrennt, das Sumpfprodukt über eine Leitung (46) im unteren Teil als Rückfluss oder Einsatz aufgegeben wird und im unteren Teil ein $C_{4-6}$-Schnitt über eine Leitung (43) als Kopfprodukt und ein $C_{5+}$-Schnitt über eine Leitung (31) als Sumpfprodukt entnommen werden.

11. Anlage nach Anspruch 9 oder 10 **gekennzeichnet durch** einen als Festbettreaktor ausgestalteten Reaktor (20) mit wenigstens zwei, vorzugsweise vier Katalysatorfestbetten (20a, 20b, 20c, 20d) und wenigstens einer Dosiereinrichtung (7, 8, 8') zur Aufteilung des Oxygenate enthaltenden Eduktstroms (O) auf Leitungen (18, 16, 16')), die jeweils vor einem der Katalysatorfestbetten (20b, 20c, 20d) münden.

12. Anlage nach einem der Ansprüche 9 bis 11, **dadurch gekennzeichnet, dass** die Rückführleitung (43) als Seitenabzug an der zweiten Trenneinrichtung (40) vorgesehen ist.

**Claims**

1. Process for the preparation of ethylene and propylene, wherein at least one oxygenate-containing reactant stream (O) and one $C_{4+}$-olefin-containing reactant stream (C) are simultaneously reacted in at least one same reactor with the same catalyst to form a product mixture (P) comprising low molecular weight olefins and gasoline hydrocarbons, **characterized in that** the ratio (V) of oxygenates in the reactant stream (O) to $C_{4+}$ olefins in the reactant stream (C) is 0.05 to 0.5, whereby the ratio (V) is calculated according to the formula

$$V = \frac{\sum_{j} k_{Oxygenat-j} * n_{Oxygenat-j}}{\sum_{i} k_{Olefin-i} * n_{Olefin-i} + \sum_{j} k_{Oxygenat-j} * n_{Oxygenat-j}}$$

with:

$k_{oxygenate-j}$ : carbon number of the oxygenate j
$n_{oxygenate\,j}$: mole current of the oxygenate j
$k_{olefin-i}$: Carbon number of olefin i
$n_{olefin-i:}$ mole current of the olefin i
and that the product mixture (P) is separated in a first separating device into a $C_3$-olefin-rich mixture (H) and a $C_{4+}$-olefin-containing stream (B), that in a second separating device the $C_{4+}$-olefin-containing stream (B) is separated into a substantially $C_4$-fraction-containing stream (F), a stream (A) rich in $C_{5+}$ gasoline hydrocarbons and a recycling stream (R) containing mainly $C_4$-$C_6$-olefins and that the recycling stream (R) is at least partially recycled to the at least one reactor, whereby the molar ratio between the recycling stream (R) and the $C_{4+}$-olefin-containing reactant stream (C) is between 0.1 and 1.5, that the second separating device is operated at a pressure of 4-15 bar and the recycling stream (R) is removed in gas form and returned directly into a line opening into the reactor.

2. Process according to claim 1, **characterized in that** the recycling stream (R) withdrawn at a side discharge.

3. Process according to one of the previous claims, **characterized in that** the oxygenate-containing reactant stream (O) is divided into several partial streams and each partial stream is passed onto one of at least two, preferably four, catalyst beds in the reactor.

4. Process according to one of the previous claims, **characterized in that** a shape-selective zeolite material, preferably an aluminosilicate of the pentasil type ZSM-5, is used as catalyst.

5. Process according to one of the previous claims, **characterized in that** the oxygenate-containing reactant stream (O) contains at least one alcohol, preferably methanol.

6. Process according to one of the previous claims, **characterized in that** the pressure at the reactor inlet is between 1.5 and 10 bar, preferably between 1.8 and 5 bar.

7. Process according to any of the previous claims, **characterized in that** the temperature at the outlet of the reactor is between 460 and 560 °C, preferably between 480 and 510 °C.

8. Process according to any of the previous claims **characterized in that** the oxygenate-containing reactant stream (O) contains at least one oxygenate which has arisen as a by-product in the production of ethanol by fermentation and/or in a Fischer-Tropsch synthesis, and/or **in that** the $C_{4+}$-olefin-containing reactant stream (C) contains at least one $C_4$ to $C_{10}$-olefin which has arisen as a primary product in a Fischer-Tropsch synthesis.

9. Plant for the production of ethylene and propylene, in particular for carrying out a process according to one of the preceding claims, with at least one catalytic reactor (20) for the simultaneous reaction of an reactant stream (O) containing at least one oxygenate and an reactant stream (C) containing at least one $C_{4+}$-olefin, into which at least one feed line (14) of the reactant current (O) and one feed line (16) of the reactant current (C) flow, **characterized by** at least one metering device (6, 7, 8, 9, 8') with which the ratio (V) of oxygenates to $C_{4+}$-olefins can be adjusted to a value between 0.05 and 0.5, whereby the ratio (V) is calculated according to the formula

$$V = \frac{\sum_{j} k_{Oxygenat-j} * n_{Oxygenat-j}}{\sum_{i} k_{Olefin-i} * n_{Olefin-i} + \sum_{j} k_{Oxygenat-j} * n_{Oxygenat-j}}$$

with:

$k_{oxygenate-j}$ : carbon number of the oxygenate j
$n_{oxygenate\,j}$: mole current of the oxygenate j
$k_{olefin-i}$: Carbon number of olefin i
$n_{olefin-i}$: mole current of the olefin i

by a first separating device (30) for separating the reaction mixture obtained in the reactor (20) into a $C_{3-}$-olefin-rich mixture and a $C_{4+}$-olefin-containing mixture, by a second separating device (40) operated at a pressure of 4-15 bar for separating the $C_{4+}$-olefin-containing mixture into a stream essentially containing $C_4$ fractions, by a stream rich in $C_{5+}$ gasoline hydrocarbons and a recycling stream (R) removed in gas form and above all containing $C_4$-$C_6$ -olefins,

by a recycling line (43) for the recycling stream (R) leading from the second separating device (40) to the reactor (20), and by a metering device (44) with which the molar ratio between the recycling stream (R) and the reactant stream (C) is adjustable to a value between 0.1 and 1.5.

**10.** Plant according to claim 9, **characterized in that** the second separating device (40) is designed in the form of an integrated column, wherein in the upper part a $C_4$ cut is separated off as top product via a line (42), the bottom product is fed in via a line (46) in the lower part as backflow or insert and in the lower part a $C_{4-6}$ cut is taken out via a line (43) as top product and a $C_{5+}$ cut via a line (31) as bottom product.

**11.** Plant according to claim 9 or 10, **characterized by** a reactor (20) designed as a fixed-bed reactor with at least two, preferably four fixed catalyst beds (20a, 20b, 20c, 20d) and at least one metering device (7, 8, 8') for distributing the oxygenate-containing reactant flow (O) to lines (18, 16, 16')) which each open upstream of one of the fixed catalyst beds (20b, 20c, 20d).

**12.** Plant according to one of claims 9 to 11, **characterized in that** the return line (43) is provided as a side discharge on the second separating device (40).

**Revendications**

**1.** Procédé de fabrication d'éthylène et de propylène, consistant à faire réagir au moins un flux de produits de départ contenant des produits d'oxydation (O) et au moins un flux de produits de départ contenant des oléfines en $C_{4+}$ (C) simultanément dans au moins un même réacteur sur un même catalyseur, pour ainsi obtenir un mélange de produits d'arrivée (P) comprenant des oléfines à faible masse moléculaire et des hydrocarbures d'essence, **caractérisé en ce que** le rapport (V) entre les produits d'oxydation (O) et les oléfines en $C_{4+}$ (C) dans le flux de produits de départ est compris entre 0,05 et 0,5, le rapport (V) étant calculé selon la formule

$$V = \frac{\sum\limits_{j} k_{Oxygenat-j} * n_{Oxygenat-j}}{\sum\limits_{i} k_{Olefin-i} * n_{Olefin-i} + \sum\limits_{j} k_{Oxygenat-j} * n_{Oxygenat-j}}$$

avec :

$k_{oxygenat-j}$ : nombre d'atomes de carbone du produit d'oxydation j
$n_{oxygenat-j}$ : flux molaire du produit d'oxydation j
$k_{Olefin-i}$ : nombre d'atomes de carbone de l'oléfine i
$n_{Olefin-i}$ : flux molaire de l'oléfine i

et que le mélange de produits d'arrivée (P) est séparé dans un premier dispositif de séparation en un mélange riche en oléfines en $C_{3-}$ (H) et un flux contenant des oléfines en $C_{4+}$ (B), que le flux contenant des oléfines en $C_{4+}$ (B) est séparé dans un deuxième dispositif de séparation en un flux (F) contenant essentiellement des fractions en $C_4$, un flux (A) riche en hydrocarbures d'essence en $C_{5+}$ et en un flux de recyclage (R) contenant surtout des oléfines en $C_4$-$C_6$, et que le flux de recyclage (R) est au moins partiellement recyclé dans l'au moins un réacteur, le rapport molaire entre le flux de recyclage (R) et le flux de produits de départ contenant des

oléfines en $C_{4+}$ (C) étant compris entre 0,1 et 1,5, et que le deuxième dispositif de séparation est mis en oeuvre à une pression comprise entre 4 et 15 bar, et le flux de recyclage (R) est prélevé sous forme gazeuse et directement recyclé dans une conduite débouchant dans le réacteur.

2. Procédé selon la revendication 1, **caractérisé en ce que** le flux de recyclage (R) est prélevé par soutirage latéral.

3. Procédé selon l'une des revendications précédentes, **caractérisé en ce que** le flux de produits de départ contenant des produits d'oxydation (O) est divisé en plusieurs flux partiels pour faire passer chacun des flux partiels sur un des lits de catalyseur dont le réacteur renferme au moins deux et au de préférence quatre.

4. Procédé selon l'une des revendications précédentes, **caractérisé en ce que** l'on utilise en tant que catalyseur une zéolithe sélective de forme, s'agissant préférentiellement d'un alumosilicate de type pentasil ZSM-5.

5. Procédé selon l'une des revendications précédentes, **caractérisé en ce que** le flux de produits de départ contenant des produits d'oxydation (O) contient au moins un alcool, de préférence du méthanol.

6. Procédé selon l'une des revendications précédentes, **caractérisé en ce qu'**à l'entrée du réacteur, la pression est comprise entre 1,5 et 10 bar, de préférence entre 1,8 et 5 bar.

7. Procédé selon l'une des revendications précédentes, **caractérisé en ce qu'**à la sortie du réacteur, la température est comprise entre 460 et 560 °C, de préférence entre 480 et 510 °C bar.

8. Procédé selon l'une des revendications précédentes, **caractérisé en ce que** le flux de produits de départ contenant des produits d'oxydation (O) contient au moins un produit d'oxydation constituant un sous-produit de la production d'éthanol par fermentation et/ou d'une synthèse Fischer-Tropsch, et/ou que le flux de produits de départ contenant des oléfines en $C_{4+}$ (C) contient au moins une oléfine en $C_4$ à $C_{10}$ constituant un produit principale d'une synthèse Fischer-Tropsch.

9. Installation qui est destinée à la fabrication d'éthylène et de propylène, notamment à la mise en oeuvre d'un procédé selon l'une des revendications précédentes, et qui comporte au moins un réacteur catalytique (20) lequel permet de faire réagir simultanément au moins un flux de produits de départ contenant des produits d'oxydation (O) et au moins un flux de produits de départ contenant des oléfines en $C_{4+}$ (C) et dans lequel débouchent au moins une conduite (14) d'introduction du flux de produits de départ (O) et une conduite (16) d'introduction du flux de produits de départ (C), **caractérisée par** au moins un dispositif de dosage (6, 7, 8, 9, 8') permettant de régler le rapport (V) entre les produits d'oxydation et les oléfines en $C_{4+}$ à une valeur comprise entre 0,05 et 0,5, le rapport V étant calculé selon la formule

$$V = \frac{\sum_{j} k_{Oxygenat-j} * n_{Oxygenat-j}}{\sum_{i} k_{Olefin-i} * n_{Olefin-i} + \sum_{j} k_{Oxygenat-j} * n_{Oxygenat-j}}$$

avec :

$k_{Oxygenat-j}$ : nombre d'atomes de carbone du produit d'oxydation j
$n_{Oxygenat-j}$ : flux molaire du produit d'oxydation j
$k_{Olefin-i}$ : nombre d'atomes de carbone de l'oléfine i
$n_{Olefin-i}$ : flux molaire de l'oléfine i
un premier dispositif de séparation (30) permettant de séparer le mélange réactionnel obtenu dans le réacteur (20) en un mélange riche en oléfines en $C_{3-}$ et un mélange contenant des oléfines en $C_{4+}$,
un deuxième dispositif de séparation (40) qui est mis en oeuvre à une pression comprise entre 4 et 15 bar et qui permet de séparer le mélange contenant des oléfines en $C_{4+}$ en un flux contenant essentiellement des fractions en $C_4$, un flux riche en hydrocarbures d'essence en $C_{5+}$ et en un flux de recyclage (R), prélevé sous forme gazeuse, contenant surtout des oléfines en $C_4$-$C_6$,
une conduite de recyclage (43) qui est destinée à un flux de recyclage (R) et qui mène du deuxième dispositif de séparation (40) au réacteur (20), et

un dispositif de dosage (44) permettant de régler le rapport molaire entre le flux de recyclage (R) et le flux de produits de départ (C) à une valeur comprise entre 0,1 et 1,5.

10. Installation selon la revendication 9, **caractérisée en ce que** le deuxième dispositif de séparation (40) est réalisé sous forme d'une colonne intégrée, une coupe en $C_4$ étant séparée dans la partie supérieure à travers une conduite (42) en tant que produit de tête, le produit de fond étant introduit dans la partie inférieure à travers une conduite (46) en tant que flux recyclé ou en tant que charge, pour récupérer dans la partie inférieure une coupe en $C_{4-6}$ à travers une conduite (43) en tant que produit de tête et une coupe en $C_{5+}$ à travers une conduite (31) en tant que produit de fond.

11. Installation selon les revendications 9 ou 10, **caractérisée par** un réacteur (20) réalisé sous forme d'un réacteur à lit fixe comportant au moins deux, de préférence quatre lits fixes de catalyseur (20a, 20b, 20c, 20d) et au moins un dispositif de dosage (7, 8, 8') destiné à répartir le flux de produits de départ contenant des produits d'oxydation (O) sur les conduites (18, 16, 16') débouchant chacune en amont de l'un des lits fixes de catalyseur (20b, 20c, 20d).

12. Installation selon l'une des revendications 9 à 11, **caractérisée en ce que** la conduite de recyclage (43) est disposée sur le deuxième dispositif de séparation (40) sous forme d'un soutirage latéral.

Fig. 1

Fig. 2

C$_4$-KW

C$_5$+Fraktion

C$_3$-Olefine

H$_2$O (lq)

H$_2$O (g)

C$_{4+}$Olefin

Oxygenat

Fig. 3

C₄-KW

C₅₊-Fraktion

C₂-Olefine

MeOH

H₂O (lq)

H₂O (g)

C₄₊-Olefin

Oxygenat

**Fig. 4**

**Fig. 5**

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005048931
- WO 2008039552 A1
- US 20040087824 A1
- DE 102007045238 A1
- EP 2058290 A1
- DE 102006026103 A1
- WO 2006048184 A1
- DE 102008058931 A1